(19) Europäisches Patentamt
European Patent Office
Office européen des brevets

(11) Veröffentlichungsnummer: **0 399 448 A2**

# (12) EUROPÄISCHE PATENTANMELDUNG

(21) Anmeldenummer: **90109667.7**

(22) Anmeldetag: **22.05.90**

(51) Int. Cl.⁵: **C07H 15/04, C12P 19/44, //(C12P19/44,C12R1:01)**

(30) Priorität: **06.07.89 DE 3922228**
**25.05.89 DE 3916956**

(43) Veröffentlichungstag der Anmeldung:
**28.11.90 Patentblatt 90/48**

(84) Benannte Vertragsstaaten:
**AT BE CH DE DK ES FR GB GR IT LI LU NL SE**

(71) Anmelder: **Verein der Zuckerindustrie**
**Am Hofgarten 8**
**D-5300 Bonn(DE)**

(72) Erfinder: **Kunz, Markwart, Dr. Dipl.-Chem.**
**Kötherberg 6**
**D-3300 Braunschweig(DE)**
Erfinder: **Matalla, Klaus, Dipl.-Chem.**
**Fasanenstr. 62**
**D-3300 Braunschweig(DE)**
Erfinder: **Stoppok, Eberhard, Dr. Dipl.-Biol.**
**Habichtweg 16**
**D-3300 Braunschweig(DE)**
Erfinder: **Rieger, Sabine, Dipl.-Chem.**
**Neuer Weg 8**
**D-3300 Braunschweig(DE)**
Erfinder: **Buchholz, Klaus, Prof. Dr.**
**Rüninger Weg 44**
**D-3300 Braunschweig(DE)**

(74) Vertreter: **Einsel, Martin et al**
**Dr.R. Döring, Dr.J. Fricke, M.Einsel**
**Jasperallee 1a**
**D-3300 Braunschweig(DE)**

## (54) Diamin.

(57) Polyhydroxydiamine werden als Saccharidderivate mit dem Vorteil verminderter Toxizität, besserer Hautverträglichkeit und biologischer Abbaubarkeit gegenüber bekannten auf petrochemischer Basis beruhenden Diaminen vorgeschlagen. Insbesondere Derivate von Sacchariden mit je einer Aminogruppe in zwei Monosaccharideinheiten, speziell Diaminodidesoxyderivate von reduzierenden Disacchariden wie Isomaltulose, Trehalulose, Maltose, Lactose und Leucrose, besitzen diese Eigenschaften.

EP 0 399 448 A2

## Diamin

Die Erfindung betrifft Diamine.

Diamine finden in der technischen Chemie Verwendung, insbesondere zur Herstellung von Polymeren. Sie eignen sich zur Umsetzung mit Carbonsäuren und ihren Derivaten sowie Isocyanaten und Sulfonsäuren, vgl. Ullmanns Enzyklopädie der Technischen Chemie, 4.Auflage, Verlag Chemie, Weinheim, Bd. 19 (1980), Seiten 41-44.

Verbreitet sind Kondensationsprodukte von Diaminen. Die Kondensation mit Epoxiden (Ullmanns Enzyklopädie, a.a.0. , Bd. 10 (1975), Seite 570) führt zu Aminen, mit Diepoxiden zu Polyaminen, mit Isocyanaten zu Harnstoffderivarten, mit Diisocyanaten zu Polyharnstoffderivaten (Ullmanns Enzyklopädie, a.a.0. , Bd. 19 (1980), Seiten 302-309), die in Polyurethanschaumstoffen, wie sie im Kunststoffsektor gebräuchlich sind, einsetzbar sind.

Heute vielfach verwendete Diamine leiten sich von Alkanen ab.

Aufgabe der Erfindung ist es demgegenüber, eine neuartige Substanz vorzuschlagen, die nicht auf einer Alkanbasis beruht. Zugleich soll ein Herstellungsverfahren für diese neuartigen Diamine beschrieben werden.

Die erfindungsgemäßen Diamine sind Polyhydrc .iamine.

Diese Diamine weisen bei der Verwendung zur Herstellung von Polymeren bzw. Polykondensationsprodukten bessere hydrophile Eigenschaften auf.

Außer der Aminfunktion können diese Polyhydroxydiamine auch mit ihren OH-Funktionen in chemischen Synthesen genutzt werden. Dadurch können die Primärprodukte, lineare Polymere, durch dreidimensionale Vernetzung mit Dicarbonsäuren zu Polyestern oder mit Diisocyanaten z.B. zu geschäumten Polyurethanen weiter umgesetzt werden. Damit lassen sich prinzipiell auch neuartige Reaktionswege und Produkte erschließen, die mit heute gebräuchlichen Diaminen nicht darstellbar sind.

Besonders bevorzugt werden Polyhydroxydiamine als Derivate eines Saccharids, insbesondere eines Oligosaccharids, mit je einer Aminogruppe in zwei Monosaccharideinheiten. Diese Saccharidderivate besitzen die Vorteile verminderter Toxizität, bessere Hautverträglichkeit und häufig biologische Abbaubarkeit.

Es besteht ein erheblicher Bedarf an Synthesebausteinen dieser Art. Sie können in zahlreichen Produkten Anwendung finden, insbesondere im Haushalt, der Kosmetik oder der Pharmazie.

Auch bei der Synthese van Tensiden können die neuartigen Polyhydroxydiamine eine Rolle spielen.

Besonders bevorzugt sind die Polyhydroxydiamine Diaminodidesoxyderivate der Isomaltulose, der Trehalulose, der Maltose, der Lactose oder der Leucrose. Alle fünf Grundstoffe stehen zunehmend großtechnisch zur Verfügung. Isomaltulose, auch unter dem Namen Palatinose (eingetragenes Warenzeichen der Fa. Südzucker) bekannt, und Trehalulose sind Oligosaccharide, die als Umlagerungsprodukte von Saccharose bei der Zuckerherstellung gewonnen werden können.

Als besonders praktikabel herstellbar haben sich Polyhydroxydiamine mix den beiden Strukturformeln erwiesen:

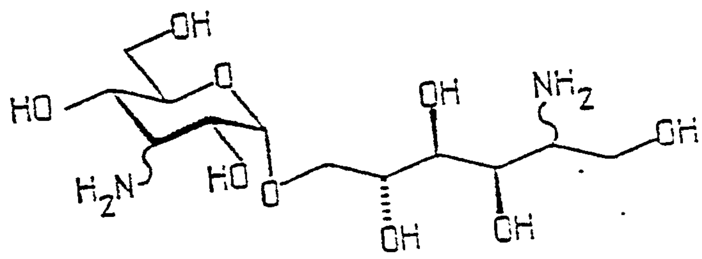

mit den vier isomeren Formen:

a) α -D-3′-amino-3′-desoxy-allopyranosyl-(1 → 6)-2-amino-2-desoxy-sorbit
b) α -D-3′-amino-3′-desoxy-allopyranosyl-(1 → 6)-2-amino-2-desoxy-mannit
c) α -D-3′-amino-3′-desoxy-glucopyranosyl-(1 → 6)-2-amino-2-desoxy-sorbit
d) α -D-3′-amino-3′-desoxy-glucopyranosyl-(1 → 6)-2-amino-2-desoxy-mannit und

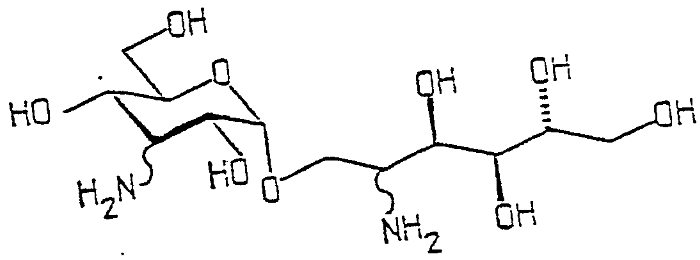

mit den vier isomeren Formen:

a) α -D-3′-amino-3′-desoxy-allopyranosyl-(1 → 1)-2-amino-2-desoxy-sorbit

b) α -D-3′-amino-3′-desoxy-allopyranosyl-(1 → 1)-2-amino-2-desoxy-mannit

c) α -D-3′-amino-3′-desoxy-glucopyranosyl-(1 → 1)-2-amino-2-desoxy-sorbit

d) α -D-3′-amino-3′-desoxy-glucopyranosyl-(1 → 1)-2-amino-2-desoxy-mannit.

Diese Substanzen können durch entsprechende Verarbeitung van Isomaltulose bzw. Trehalulose gewonnen werden.

Als Zwischenprodukte bei der Herstellung dieser beiden Chemikalien entstehen ebenfalls neue Substanzen, die auch stabil sind und beispielsweise durch Lyophilisation (Gefriertrocknung) als Feststoff gewonnen werden können. Es handelt sich um 3′-Oxo-Isomaltulose und 3′-Oxo-Trehalulose.

Durch die Hydrophilie der Zuckerbausteine sind die genannten Produkte hautfreundlicher als petrochemische Produkte.

Monoamine von Disacchariden eignen sich dagegen nicht für die Herstellung von Polymeren und anderen Stoffen, deren Herstellung eine doppelte Aminfunktion erfordern.

Auch aus Maltose und Lactose (ebenfalls beides Disaccharide) konnten Diaminodidesoxyderivate gewonnen werden, bevorzugt mix der Strukturformel:

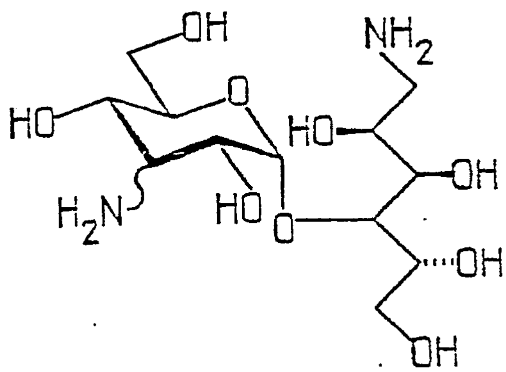

und den beiden isomeren Formen:

a) α -D-3′-amino-3′-desoxy-allopyranosyl-(1 → 4)-1-amino-1-desoxy-sorbit

b) α -D-3′-amino-3′-desoxy-glucopyranosyl-(1 → 4)-1-amino-1-desoxy-sorbit bzw. mix der Strukturformel:

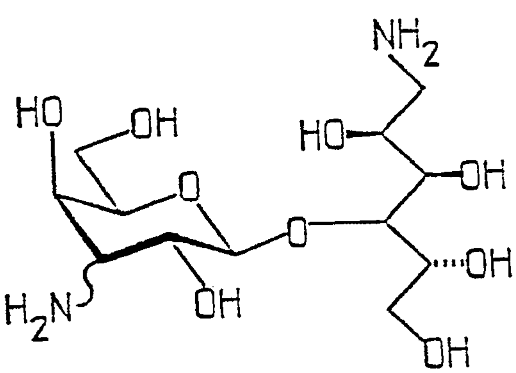

und den beiden isomeren Formen:

a) β -D-3′-amino-3′-desoxy-galactopyranosyl-(1 → 4)-1-amino-1-desoxy-sorbit

b) β -D-3′-amino-3′-desoxy-gulopyranosyl-(1 → 4)-1-amino-1-desoxy-sorbit.

Aus der DE 36 25 931 A1 sind verschiedene Aminopolyole bekannt. Diese werden durch reduktive Aminierung aus den Dissachariden Isomaltose, Isomaltulose und α -D-Glucopyranosyl (1 → 1) D-fructose hergestellt. Die reduktive Aminierung erfolgt mittels Ammoniak oder Hydrazinverbindungen in Gegenwart von Katalysatoren, wie Raney-Nickel. Eine Weiterverarbeitung zur Herstellung von Polymeren wird als Möglichkeit pauschal erwähnt, wobei eine Verwendung als Dickungsmittel in der Technik, Pharma- und Nahrungsmittelindustrie vorgeschlagen wird.

Gemäß diesem Stand der Technik wird ein monofunktioneller Baustein mit einer polymerisationsfähigen Gruppe verknüpft. Zusätzlich muß eine Vinylgruppe eingeführt werden. Eine Polyaddition ader Polykondensation ist ein grundsätzlich anderer Vorgang. Aus dem Stand der Technik ist weder ein Hinweis auf Diamine überhaupt noch auf die erfindungsgemäßen Diamine und auch kein Hinweis auf ein Verfahren zur Herstellung derartiger Polyhydroxydiamine zu entnehmen.

Als Verfahren für die Herstellung der Polyhydroxydiamine werden folgende Schritte vorgeschlagen:

a) Zellen des Stammes Agrobacterium tumefaciens (NCPPB 396) werden durch Fermentation gewonnen;

b) Ein Oligosaccharid wird mix den Zellen inkubiert;

c) Aus dem gebildeten Produkt werden Polysaccharide und Proteine ausgefällt und entfernt;

d) Das Reaktionsprodukt wird anschließend mittels Aminierungsmitteln reduktiv aminiert;

e) Aufarbeitung und Trennung des entstehenden Produktgemisches und Isolierung der darin enthaltenen Polyhydroxydiamine.

Insbesondere Hydrazin hat sich als Aminierungsmittel bewährt, speziell bei Verwendung eines Katalysators, insbesondere Raney-Nickel.

Die Verwendbarkeit von Zellen des Stammes Agrobacterium tumefaciens (NCPPB 396) zur Oxidation von einzelnen Disacchariden ist von DE LEY 1958 entdeckt worden (BERNAERTS, M.J.; DE LEY, J., in: J. Gen. Microbiol. 22 (1960), 129-36; VAN BEEUMEN. J.; DE LEY, J., in: European J. Biochem. 6 (1968), 331).

Aus dem letztgenannten Aufsatz ist in Übereinstimmung mit den Angaben von Fukui und Hayano (HAYANO, K.; FUKUI, S., in: J. Biol. Chem. 242 (1967), 3365-3372) zu entnehmen, welche Anforderungen an die Konfiguration und Konformation des Oligosaccharids gestellt werden können, damit es durch Zellen des Stammes Agrobacterium tumefaciens (NCPPB 396) günstig angegriffen werden kann. Ein besonders bevorzugt nach dem o. g. Verfahren herstellbares Polyhydroxydiamin ist danach dadurch gekennzeichnet, daß der endständige nicht reduzierende Glycosylrest eine Aldo-hexopyranose ist, daß am Kohlenstoffatom C 6 eine freie $CH_2OH$-Gruppe vorhanden ist, daß am Kohlenstoffatom C 4 eine unsubstituierte OH-Gruppe vorliegt, und daß unter der Voraussetzung einer C1-Sesselkonormation am Kohlenstoffatom C 3 eine äquatoriale Anordnung der OH-Gruppe vorliegt.

Die verschiedenen Kriterien sind dabei in den beiden genannten Aufsätzen eher theoretisch und im Hinblick auf gänzlich andere Reaktionen hin aufgestellt worden.

Die Entstehung von 3′-Oxo-Disacchariden als technisches Zwischenprodukt wurde jedoch nicht beschrieben. Insbesondere die Oxidation von Isomaltulose und Trehalulose ist bisher noch nicht durchgeführt worden.

Unbefriedigend war bisher auch die Vermehrung des Stammes Agrobacterium tumefaciens (NCPPB 396). Hier hat sich der Einsatz eines Hefeextrakts (Fa. Merck, Nr. 3753) in der Nährlösung als überraschend erfolgreich erwiesen. Dieser an sich bekannte Hefeextrakt war bei der Vermehrung des Stammes Agrobacterium tumefaciens (NCPPB 396) im Stand der Technik noch nicht eingesetzt worden.

Als Nährlösung für den Stamm Agrobacterium tumefaciens (NCPPB 396) wird ein Medium mit folgenden Bestandteilen vorgeschlagen:

| | |
|---|---|
| Hefeextrakt (Fa. Merck, Nr. 3753) | 0,5 g/l |
| Harnstoff | 0,9 g/l |
| Zitronensäure | 0,15 g/l |
| $FeSO_4 \times 2H_2O$ | 0,01 g/l |
| $MgSO_4 \times 2H_2O$ | 0,15 g/l |
| $CaCl_2 \times 2H_2O$ | 0,025 g/l |
| Saccharose | 20,0 g/l |
| $Na_2HPO_4 \times 2H_2O$ | 10,8 g/l |
| $KH_2PO_4$ | 5,35 g/l |

Mit diesem Nährmedium wurde ein deutlich besseres Wachstum für den Stamm Agrobacterium tumefaciens (NCPPB 396) festgestellt im Vergleich zu bekannten Nährmedien.

Im folgenden wird ein Verfahren zur Herstellung eines Polyhydroxydiamins auf Isomaltulosebasis beschrieben. Dieses Verfahren hat sich praktisch bewährt und führt zu einer brauchbaren Ausbeute. Zunächst wird eine Vermehrung des Stammes Agrobacterium tumefaciens (NCPPB 396) durchgeführt derart, daß dabei eine besondere Bereitschaft des Stammes für die anschließend beabsichtigte Fermentation bzw. Oxidation von Oligosacchariden entsteht.

Zu diesem Zweck wird der Stamm Agrobacterium tumefaciens (NCPPB 396) zunächst auf Agarmedium (5,0 g Pepton, 3,0 g Fleischestrakt, 15,0 g Agar, 1000 ml deionisiertes Wasser) als Schrägkultur geführt.

Mittels einer Platinöse wird von Agarröhrchen auf 100 ml der obengenannten Nährlösung (Medium 1) in 500 ml-Erlenmeyerkolben mit zwei gegenüberliegenden Schikanen übergeimpft, wodurch eine Vorkultur 1 entsteht.

Die Vorkultur 1 wird im Schüttelschrank 40 Stunden bei 28°C bebrütet. Mit 5 ml der entstehenden Zellsuspension werden 500 ml der Nährlösung Medium 1 in 2000 ml Erlenmeyerkolben mix Schikanen beimpft. Es entsteht die Vorkultur 2.

Die Vorkultur 2 wird anschließend wiederum bei 28°C im Schüttelschrank für 20 Stunden bebrütet. Danach werden mit zwei Kolben der Vorkultur 2 mix Gesamtvolumen von 1 l 12 l Nährlösung (Medium 1) in einem 15 l -Laborfermenter beimpft. Anschließend wird bei 28°C und einer Rührgeschwindigkeit von 400 $min^{-1}$ fermentiert. Dabei wird ein Rührerdurchmesser von 7 cm eingesetzt. Bei anderen Rührerdurchmesser sind dementsprechend andere Rührgeschwindigkeiten zu wählen. Die Belüftungsrate wird nach 4 Stunden Fermentationszeit von 1,6 l $min^{-1}$ auf 6,5 l $min^{-1}$ erhöht.

Die Fermentation wird nach 18 Stunden abgebrochen und die Biomasse aus der Fermenterbrühe durch Zentrifugation gewonnen. Nach zweimaligem Waschen mit physiologischer Saline können die geernteten Zellen als ruhende Zellen zur Stoffumwandlung eingesetzt werden. Die Zellausbeute beträgt ca. 2,2 g Trockensubstanz pro l.

In einem 500 ml-Kolben mit Schikanen werden 0,9 g (bezogen auf die Trockensubstanz) Zellen in 100 ml deionisiertem Wasser suspendiert. Die Zellen werden also als sogenannte "Freie Zellen" eingesetzt. Nun werden 2,5 g Isomaltulose hinzugegeben und bei 28°C im Schüttelschrank inkubiert.

Die Substratabnahme (Abnahme des Isomaltulose-Anteils) sowie die Produktbildung können über Hochdruckflüssigkeitschromatographie verfolgt werden. Nach ca. 20 Stunden ist die gesamte Isomaltulose verbraucht. Dies macht sich dadurch bemerkbar, daß im Chromatogramm nur noch ein einziger Peak erscheint, wodurch ein selektiver Reaktionsverlauf angezeigt wird.

Die Biomasse wird nun abzentrifugiert. Mit dem 10fachen Volumen Methanol werden Polysaccharide und Proteine ausgefällt und durch Zentrifugation und Membranfiltration (200 nm) entfernt. Das Filtrat wird schonend auf ca. 1/10 seines Volumens eingeengt, und das Reaktionsprodukt wird durch Lyophilisation (Gefriertrocknung) als Feststoff gewonnen. Die Ausbeute beträgt ca. 50% bis 60%. Das Reaktionsprodukt wird mittels $^{13}C$-NMR-Spektroskopie als 3′-Oxo-Isomaltulose identifiziert.

Dieses Zwischenprodukt wird jetzt reduktiv aminiert. Hierzu werden zunächst in einem 1 l-Rundkolben 100 g (0,29 mol) 3′-Oxo-Isomaltulose in 500 ml deionisiertem Wasser gelöst, dann mit 60 ml einer 80%igen Hydrazin ($H_2N$-$NH_2$)-Lösung versetzt und schließlich bei Raumtemperatur für 18 Stunden gerührt.

Zusammen mit 25 g feuchtem Raney-Nickel-Katalysator wird diese Lösung in einen 1-l-Hochdruckautoklaven gegeben, der vor der Reaktion durch dreimaliges Spülen mit Wasserstoff (50 bar) sauerstofffrei gemacht wird.

Bei 100-200 bar Wasserstoffdruck (bevorzugt 150 bar), 30-65°C Temperatur (bevorzugt 50°C) und einer Rührgeschwindigkeit von 1000 $min^{-1}$ wird anschließend reduktiv aminiert (Rührerdurchmesser hier 5 cm, bei anderen Rührerdurchmessern wird entsprechend die Rührgeschwindigkeit angepaßt. Entscheidend

ist die gute Durchmischung der Reaktanden) . Die Temperatur wird nach Abklingen der anfangs auftretenden Reaktionswärme auf 50°C gehalten. Ähnliche Schritte sind für die reduktive Aminierung anderer Substanzen von Ali-Reza Haji Begli, Dissertation Technische Universität Braunschweig 1988, vorgeschlagen worden.

Nach 24 Stunden wird der feste Katalysator abfiltriert und die wässerige Phase zur Entfernung überschüssigen Hydrazins am Rototiansverdampfer eingeengt.

Die Aufarbeitung des Produktgemisches geschieht nun mittels. Chromatographie an einem Kationenaustauscherharz. Es wird eine temperierbare Glassäule (Länge 100 cm, Durchmesser 5 cm) verwendet, die mit dem Harz (z.B. CG 120 II, Fa. Serva) in der $NN_4$+-Form gefüllt ist.

Durch Variation in der Ammoniakkonzentration des Fließmittels können die Produktsubstanzen hinreichend voneinander getrennt werden. Als Produktsubstanzen sind dabei unterschiedlich aminierte Mono- und Disaccharide vorhanden. Diese Produkte entstehen dadurch, daß zum einen Disaccharide lediglich einfach aminiert wurden (Produkt: Monoamin des Disaccharids), daß ein Disaccharid zweifach aminiert wurde (Produkt: Diamin des Disaccharids) und dadurch, daß zweifach aminierte Disaccharide instabil wurden und in zwei Monosaccharide zerfallen sind.

Folgende Elutionsreihenfolge der möglichen Produktsubstanzen wird gefunden:

1. Neutralsubstanzen
2. Monoamine der Disaccharide
3. Monoamine der Monosaccharide (aminiert am sekundären C-Atom)
4. Diamine der Disaccharide
5. Monoamine der Monosaccharide (aminiert am primären C-Atom).

Dabei wurden folgende Standardchromatographiebedingungen eingesetzt:

| | |
|---|---|
| Fließmittel | 0,1 - 0,5% Ammoniaklösung |
| Fluß | 10 - 50 ml/min |
| Temperatur | 20 - 40°C |
| Detektion | Differentialrefraktometer. |

Bezogen auf das eingesetzte 3′-Oxo-Disaccharid, hier also die 3′-Oxo-Isomaltulose, werden Diamine der Disaccharide mit einer Ausbeute von ca. 50% erhalten.

Anstelle der obenerwähnten "Freien Zellen" können im Verfahren auch "immobilisierte Zellen" bei der Durchführung der Oxidationsreaktion eingesetzt werden (vgl. Klaus Dieter Vorlop, Dissertation Technische Universität Braunschweig 1984). Zellen von Agrobacterium tumefaciens werden in Alginat immobilisiert und die erhaltenen Perlen (Durchmesser 0,5-2 mm) zur Produktion von 3′-Oxo-Isomaltulose eingesetzt. Die Umsätze in praktischen Versuchen erreichten 40-60% der mit freien Zellen erzielten Werte.

Der Vorteil liegt demgegenüber in der erleichterten Rückgewinnbarkeit der Zellen in den Perlen. Nach Art eines sich nur langsam erschöpfenden Katalysators können sie immer wieder erneut eingesetzt werden.

Als weiteres Beispiel wird praktisch das gleiche Verfahren eingesetzt, lediglich wird anstelle der Isomaltulose 2,5 g Trehalulose eingesetzt. Trehalulose wird auch als α-D-Glucopyranosyl-(1 → 1)-D-Fructose bezeichnet. Als Zwischenprodukt entsteht in diesem Falle 3′-Oxo-Trehalulose. Die Weiterverarbeitung erfolgt ebenfalls wie im 1.Beispiel.

Entsprechend erfolgt das Verfahren auch bei Lactose, Maltose und Leucrose. Als Zwischenprodukte entstehen in diesem Fall 3′-Oxo-Lactose bzw. 3′-Oxo-Maltose bzw. 3′-Oxo-Leucrose.

Auch Multooligosaccharide, Stärkehydrolysate oder Raffinose sind zur Gewinnung der erfindungsgemäßen Polyhydroxydiamine denkbar.

## Ansprüche

1. Diamin, nämlich Polyhydroxydiamin.

2. Diamin nach Anspruch 1 als Derivat eines Saccharids.

3. Diamin nach Anspruch 2, **dadurch gekennzeichnet,** daß das Saccharid ein Oligosaccharid mit je einer Aminogruppe in zwei Monosaccharideinheiten ist.

4. Diamin nach Anspruch 3, **dadurch gekennzeichnet,** daß der endständige nicht reduzierende Glycosylrest eine Aldo-hexopyranose ist, daß am Kohlenstoffatom C 6 eine freie $CH_2OH$-Gruppe vorhanden ist, daß am Kohlenstoffatom C 4 eine unsubstituierte OH-Gruppe vorliegt, und daß unter der Voraussetzung

einer C1-Sesselkonformation am Kohlenstoffatom C 3 eine äquatoriale Anordnung der OH-Gruppe vorliegt.

5. Diamin nach Anspruch 3 oder 4, **dadurch gekennzeichnet,** daß das Oligosaccharid ein reduzierendes Disaccharid ist.

6. Diamin nach Anspruch 5, **dadurch gekennzeichnet,** daß es ein Diaminodidesoxyderivat der Isomaltulose ist.

7. Diamin nach einem der vorstehenden Ansprüche, **gekennzeichnet durch** die Strukturformel:

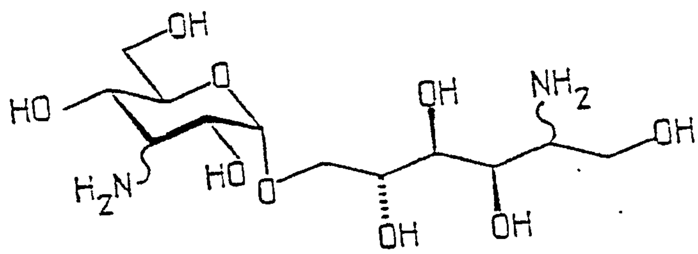

oder eine der vier isomeren Formen:

a) α -D-3′-amino-3′-desoxy-allopyranosyl-(1 → 6)-2-amino-2-desoxy-sorbit
b) α -D-3′-amino-3′-desoxy-allopyranosyl-(1 → 6)-2-amino-2-desoxy-mannit
c) α -D-3′-amino-3′-desoxy-glucopyranosyl-(1 → 6)-2-amino-2-desoxy-sorbit
d) α -D-3′-amino-3′-desoxy-glucopyranosyl-(1 → 6)-2-amino-2-desoxy-mannit.

8. Diamin nach Anspruch 5, **dadurch gekennzeichnet,** daß es ein Diaminodidesoxyderivat der Trehalulose ist.

9. Diamin nach einem der vorstehenden Ansprüche, **gekennzeichnet durch** die Strukturformel:

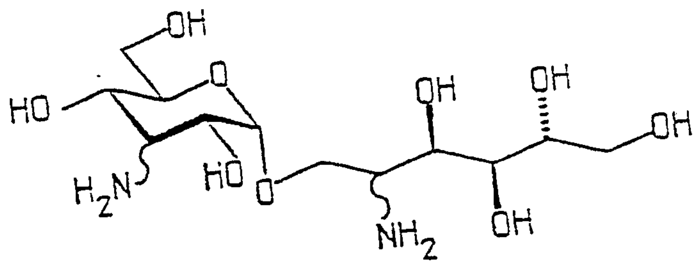

oder eine der vier isomeren Formen:

a) α -D-3′-amino-3′-desoxy-allopyranosyl-(1 → 1)-2-amino-2-desoxy-sorbit
b) α -D-3′-amino-3′-desoxy-allopyranosyl-(1 → 1)-2-amino-2-desoxy-mannit
c) α -D-3′-amino-3′-desoxy-glucopyranosyl-(1 → 1)-2-amino-2-desoxy-sorbit
d) α -D3′-amino-3′-desoxy-glucopyranosyl-(1 → 1)-2-amino-2-desoxy-mannit.

10. Diamin nach Anspruch 5, **dadurch gekennzeichnet,** daß es ein Diaminodidesoxyderivat der Maltose ist.

11. Diamin nach einem der vorstehenden Ansprüche, **ge kennzeichnet durch** die Strukturformel:

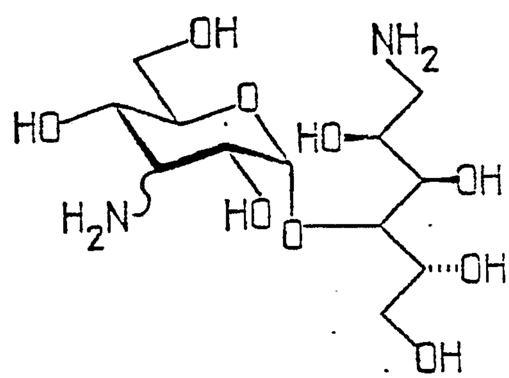

oder der eine der beiden isomeren Formen:

a) α -D-3′-amino-3′-desoxy-allopyranosyl-(1 → 4)-1-amino-1-desoxy-sorbit

b) α -D-3′-amino-3′-desoxy-glucopyranosyl (1 → 4)-1-amino-1-desoxy-sorbit.

12. Diamin nach Anspruch 5, **dadurch gekennzeichnet,** daß es ein Diaminodidesoxyderivat der Lactose ist.

13. Diamin nach einem der vorstehenden Ansprüche, **gekennzeichnet durch** die Strukturformel:

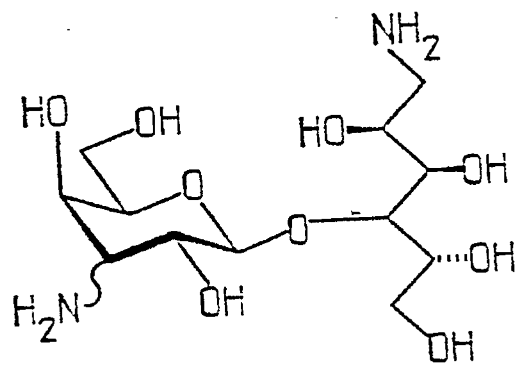

oder eine der beiden isomeren Formen:

a) β -D-3′-amino-3′-desoxy-galactopyranosyl-(1 → 4)-1-amino-1-desoxy-sorbit

b) β -D-3′-amino-3′-desoxy-gulopyranosyl-(1 → 4)-1-amino-1-desoxy-sorbit.

14. Diamin nach Anspruch 5, **dadurch gekennzeichnet,** daß es ein Diaminodidesoxyderivat der Leucrose ist.

15. Keton, nämlich 3′-Oxo-Isomaltulose.

16. Keton, nämlich 3′-Oxo-Trehalulose.

17. Verfahren zur Herstellung von Polyhydroxydiaminen mit folgenden Schritten:

a) Zellen des Stammes Agrobacterium tumefaciens (NCPPB 396) werden durch Fermentation gewonnen;

b) Ein Oligosaccharid wird mit den Zellen inkubiert;

c) Aus dem gebildeten Produkt werden Polysaccharide und Proteine ausgefällt und entfernt;

d) das Reaktionsprodukt wird anschließend mittels Aminierungsmitteln reduktiv aminiert;

e) Aufarbeitung und Trennung des entstehenden Produktgemisches und Isolierung der darin enthaltenen Polyhydroxydiamine.

18. Verfahren nach Anspruch 17, **dadurch gekennzeichnet,** daß als Aminierungsmittel Hydrazin verwendet wird und Katalysatoren, insbesondere Raney-Nickel, eingesetzt werden.

19. Verfahren nach Anspruch 17, **dadurch gekennzeichnet,** daß als Aminierungsmittel Ammoniak, Hydroxylamin oder deren Salze und Katalysatoren, insbesondere Raney-Nickel, eingesetzt werden.

20. Verfahren nach einem der Ansprüche 17 bis 19, **dadurch gekennzeichnet,** daß im Schritt e) zur Abtrennung der Polyhydroxydiamine Chromatographie eingesetzt wird.

21. Verfahren nach einem der Ansprüche 17 bis 20, **dadurch gekennzeichnet,** daß zur Vermehrung des Stammes Agrobacterium tumefaciens (NCPPB 396) ein Nährmedium eingesetzt wird, das Hefeextrakt (Fa. Merck, Nr. 3753) enthält.

22. Verfahren nach Anspruch 21, **dadurch gekennzeichnet,** daß zur Vermehrung des Stammes Agrobacterium tumefaciens (NCPPB 396) ein Nahrmedium mit:

| | |
|---|---|
| Hefeextrakt (Fa. Merck, Nr. 3753) | 0,5 g/l |
| Harnstoff | 0,9 g/l |
| Zitronensäure | 0,15 g/l |
| $FeSO_4$ x $2H_2O$ | 0,01 g/l |
| $MgSO_4$ x $2H_2O$ | 0,15 g/l |
| $CaCl_2$ x $2H_2O$ | 0,025 g/l |
| Saccharose | 20,0 g/l |
| $Na_2HPO_4$ x $2H_2O$ | 10,8 g/l |
| $KH_2PO_4$ | 5,35 g/l |

eingesetzt wird.